Europäisches Patentamt

European Patent Office

Office européen des brevets

(11) Publication number: **0 375 862 B1**

# EUROPEAN PATENT SPECIFICATION

(45) Date of publication of patent specification: **17.08.94** (51) Int. Cl.⁵: **C09J 7/02**, A61F 13/58, B32B 27/06

(21) Application number: **89119273.4**

(22) Date of filing: **17.10.89**

(54) Adhesive tapes for medical or sanitary use.

(30) Priority: **18.10.88 JP 135859/88**
**06.04.89 JP 41040/89**
**26.09.89 JP 249521/89**

(43) Date of publication of application:
**04.07.90 Bulletin 90/27**

(45) Publication of the grant of the patent:
**17.08.94 Bulletin 94/33**

(84) Designated Contracting States:
**BE DE FR GB**

(56) References cited:
DE-A- 2 830 536
US-A- 3 716 437
US-A- 3 908 650

(73) Proprietor: **NITTO DENKO CORPORATION**
**1-2, Shimohozumi 1-chome**
**Ibaraki-shi**
**Osaka (JP)**

(72) Inventor: **Arakawa, Masaaki**
**c/o Nitto Denko Corporation**
**1-2, Shimohozumi**
**1-chome Ibaraki-shi Osaka (JP)**
Inventor: **Murata, Hidehiko**
**c/o Nitto Denko Corporation**
**1-2, Shimohozumi**
**1-chome Ibaraki-shi Osaka (JP)**
Inventor: **Moriyama, Takaaki**
**c/o Nitto Denko Corporation**
**1-2, Shimohozumi**
**1-chome Ibaraki-shi Osaka (JP)**
Inventor: **Suenaga, Kazuo**
**c/o Nitto Denko Corporation**
**1-2, Shimohozumi**
**1-chome Ibaraki-shi Osaka (JP)**

(74) Representative: **Patentanwälte Grünecker,**
**Kinkeldey, Stockmair & Partner**
**Maximilianstrasse 58**
**D-80538 München (DE)**

**Description**

The present invention relates to adhesive tapes for medical or sanitary use and the like and more particularly, to adhesive tapes which can be advantageously used as fastener fixing tapes for paper diapers or as non-slip tapes for sanitary napkins, etc.

Adhesive tapes for medical or sanitary use, etc. are used in contact with human body. Therefore, feeling and softness are demanded so as not to cause redness, rash, lacerated wound, etc. to the skin.

In response to such demand, various improvements have been attempted to make on tape supports. For example, there is known an adhesive tape using as a tape support a film composed of a mixture of one or more members selected from ethylene-vinyl acetate copolymer, polyethylene or ethylene-propylene copolymer and polypropylene (Japanese Patent Application Laid-Open No. 63-112704).

However, such a tape is insufficient for redness, though occurrence of rash or lacerated would is improved. In addition, since processing characteristics upon preparation are also taken into account, the tape involves a defect that it is rough to the touch.

US-A-3716437 discloses a base film suitable for adhesive tapes of enhanced tensile strength combined with high dielectric strength formed by selecting an extruded composite film comprising superimposed films of different softening points, and then reinforcing the lower-softening film with an unwoven and unspun array of textile-length fibers. The primary field of application for these adhesive tapes is the use as cable wrapping tapes because of their electrical insulating properties.

The object of the present invention is to solve the above-mentioned defects and to provide an adhesive tape for medical or sanitary use, etc. which is endowed with properties of both softness and elasticity, gentle to the skin and excellent in handling and processing characteristics.

This object has been accomplished by a medical or sanitary adhesive tape comprising a support of a laminate structure composed of a first layer containing a thermoplastic elastomer and a second layer comprising a plastic film or nonwoven fabric, and an adhesive layer formed on at least one surface of said support, characterized in that a crosswise compression strength of said support is not greater than 200 g/mm$^2$ and said thermoplastic elastomer used in the first layer has a permanent compression strain of 5 to 100% and a Shore hardness of not less than A20 and not more than D70.

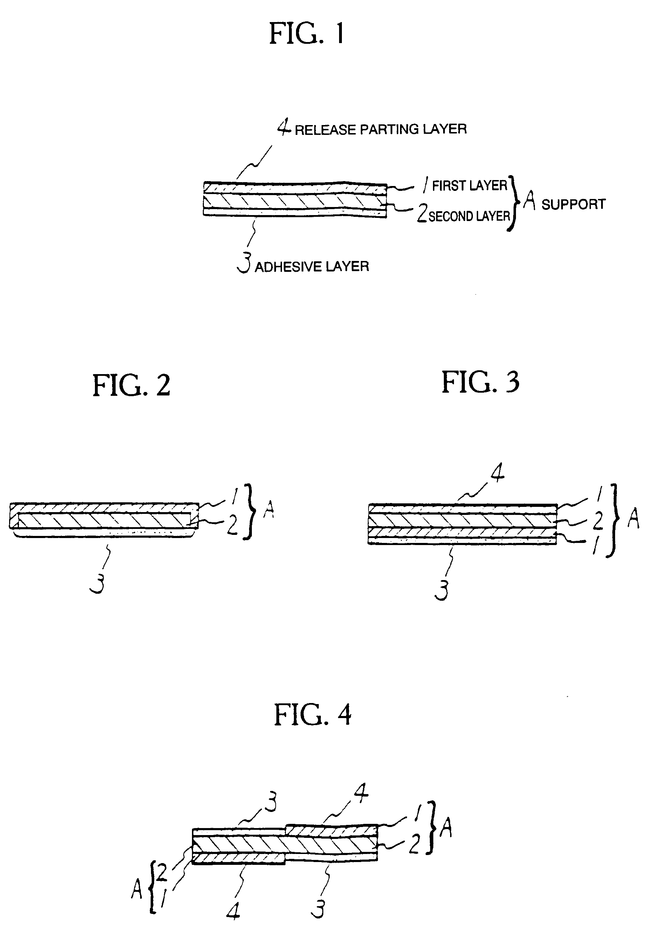

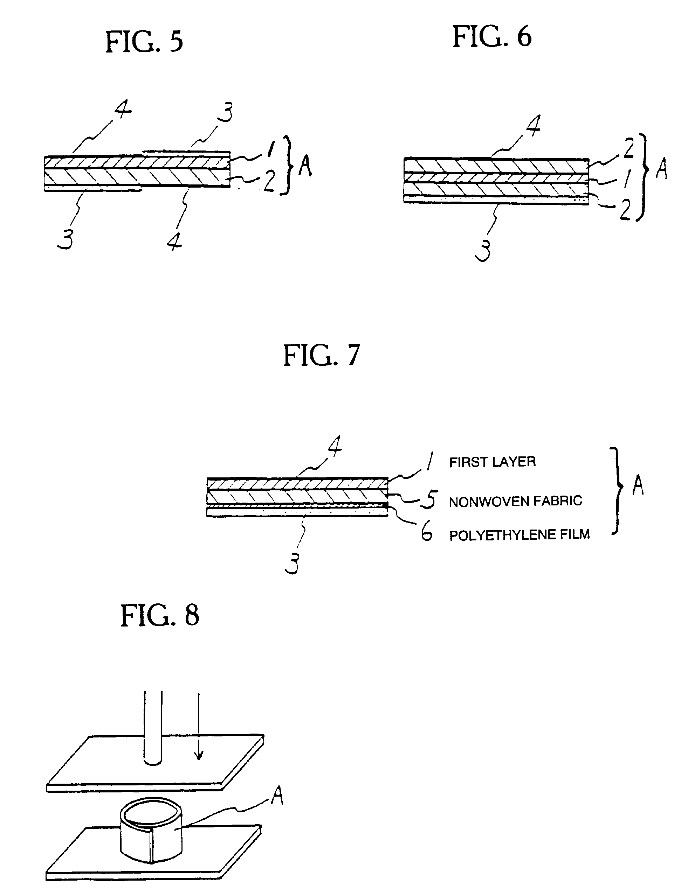

Figs. 1 through 7 are cross sectional views showing working examples of the adhesive tape according to the present invention. Fig. 8 is an explanatory illustration showing a method for determining crosswise compression strength of the support used in the present invention.

A     support
1     first layer
2     second layer
3     adhesive layer

The first layer which constitutes the support used in the present invention is an elastic film composed of thermoplastic elastomer or mainly composed of the elastomer. The thermoplastic elastomer has a permanent compression strain of 5 to 100%, preferably 20 to 60% and a Shore hardness of not less than A20 and not more than D70, preferably A40 to A90. The permanent compression strain as used herein refers to that measured at compressibility of 25% and 70 °C for 22 hours in accordance with JIS K6301. The Shore hardness is determined in accordance with ASTM D2240. By setting the values of permanent compression strain and Shore hardness in the ranges described above in the present invention, softness and elasticity of the desired adhesive tape can be obtained.

Specific examples of the thermoplastic elastomer described above include polyolefin type elastomer in which hard segment is polyethylene, polypropylene, etc. and soft segment is ethylenepropylene diene monomer, ethylenepropylene monomer, etc.; polystyrene type elastomer in which hard segment is polystyrene and soft segment is butadiene rubber, isoprene rubber, hydrogenated butadiene rubber, etc.; polyvinyl chloride type elastomer in which hard segment is polyvinyl chloride, etc. and soft segment is a plasticizer such as 2-ethylhexyl phthalate (dioctyl phthalate), etc. or a polymer blend such as NBR, partially crosslinked NBR, etc., rubber such as modified PVC, etc.; polyester type elastomer in which hard segment is polyester and soft segment is polyether or polyester; polyurethane type elastomer in which hard segment is polyurethane and soft segment is polyester or polyether; chlorinated polyeth ylene type elastomer in which hard segment is block type chlorinated polyethylene and soft segment is random chlorinated polyethylene, etc. These elastomers may be used singly or as admixture of two or more.

In addition, by using as the aforesaid first layer an elastic film comprising a mixture of the thermoplastic elastomer described above and one or more thermoplastic resins selected from polyethylene, polypropylene and ethylene-vinyl acetate copolymer, adhesiveness to the second layer which will be later described can also be more improved. As the ethylene-vinyl acetate copolymer, the copolymer having a vinyl acetate content of 5% or less is generally preferably used. The thermoplastic resin as used herein refers to crystalline resin having a storage elastic modulus G' of $10^8$ or more at normal temperature (20°C) which does not show rubber elasticity at normal temperature (20°C).

A weight ratio of the thermoplastic elastomer to the thermoplastic resin in this case is generally in the range of 1 : 9 to 9 : 1, preferably 5 : 5 to 1 : 9.

In the present invention, it is particularly preferred to use as the first layer the hydrogenated product of block copolymer represented by general formula:

A - (B - A)n

or a mixture of the block copolymer with polyolefin type thermoplastic resin such as polyethylene, polypropylene, etc.

In the formula, A is a polymer block of monovinyl-substituted aromatic hydrocarbon such as polystyrene, poly-$\alpha$-methylstyrene, etc.; B is an elastomeric polymer block of conjugated diene such as butadiene, isoprene, etc.; n is 1 to 4; and a weight ratio of A to B is 10/90 to 50/50. A weight average molecular weight of this hydrogenated block copolymer is approximately 30,000 to 300,000 and a hydrogenated amount is approximately 70 to 95% based on the diene component (component B).

For hydrogenation of the conjugated diene polymer, there is a method which comprises dissolving the polymer in an inert hydrocarbon solvent, e.g., cyclohexane, etc., adding cobalt, nickel or the like reduced using a catalyst such as an alkyl alminum, etc. to the solution, and reacting for about 10 to about 60 minutes generally at 25 to 50°C under pressure of, e.g., 5 to 40 kg/cm$^2$ hydrogen.

Further in the case of using the mixture of this hydrogenated block copolymer and polyolefin type thermoplastic resin, it is preferred to use 5 to 100 parts by weight of the polyolefin type thermoplastic resin based on 100 parts by weight of the hydrogenated block copolymer.

As the second layer in the present invention, there is used a plastic film composed of a mixture of one or more thermoplastic resins selected from polyethylene, ethylene-propylene copolymer or ethylene-vinyl acetate copolymer and polypropylene or polypropylene alone. In this case, it is desired that a weight ratio of the thermoplastic resin to polypropylene be in the range of 0 : 10 to 9 : 1, preferably 4 : 6 to 6 : 4. The thermoplastic resin as used herein refers to crystalline resin having a storage elastic modulus G' of $10^8$ or more at normal temperature (20°C) which does not show rubber elasticity at normal temperature (20°C).

As the second layer, nonwoven fabric of polyester type, nylon type or polyolefin type may also be used. In order to impart elasticity thereto, it is preferred to use nonwoven fabric having a weight of at least 20 g/m$^2$. Pressure or heat may also be applied to such nonwoven fabric to further improve its strength.

In order to firmly bind to the first layer and form the support endowed with softness and elasticity, such second layer has tough and elasticity properties so that it can reinforce the first layer without injuring softness of the first layer.

A polyethylene film of approximately 1 to 100 $\mu$m may further be laminated onto one surface or both surfaces of such nonwoven fabric, whereby the thermoplastic elastomer in the first layer and the adhesive later described can be prevented to transfer.

The support used in the present invention takes a laminate structure of the first layer and the second layer described above, that is, a dual layer structure, three layered structure or more. A thickness of the support is not particularly limited but generally from 10 $\mu$m to 1 mm. A thickness ratio of the first layer to the second layer is generally from 1 : 1 to 1000.

The adhesive layer provided at least one surface of the support described above is not particularly limited and ordinary adhesives of acrylic type, rubber type, styrene type, etc. can be used. Its thickness is set at approximately 5 to 500 $\mu$m. It is preferred that such an adhesive layer be provided on the second layer side of the support. By taking such a structure, the first layer is located on the surface and in contact with the skin upon use of the tape so that the tape is excellent in the touch.

Furthermore, a back treating agent of a long chain alkyl type or silicone type can also be applied to at least the other surface of the support.

The adhesive tape of the present invention has a crosswise compression strength of 200 g/mm$^2$ or less, preferably 40 to 100 g/mm$^2$. By setting the crosswise compression strength within such a range, the resulting adhesive tape advantageously possesses appropriate softness and elasticity. The crosswise compression strength as used herein is determined by the method which will be explained in the examples later described.

Fig. 1 is a cross sectional view of an example showing the adhesive tape of the present invention, wherein A is a support composed of first layer 1 and second layer 2 laminated therebeneath; adhesive layer 3 is provided on the side of such second layer 2; and 4 is release parting agent provided on the other surface of the support A, if necessary and desired.

Figs. 2 through 7 show cross sectional views showing other structures of the adhesive tape of the present invention, respectively.

Fig. 2 shows a structure in which first layer 1 in Fig. 1 covers second layer 2 to the side surface of second layer 2. In this case, the tape is advantageous in that the side surface of second layer 2 is not in direct contact with the skin.

Fig. 3 shows an adhesive layer using support A composed of first layer 1 having laminated second layer 2 on both surfaces thereof.

Fig. 4 shows a structure, almost the half of the tape takes the same structure as in Fig. 1 and the other half takes a structure that adhesive layer 3 is provided on support A obtained by laminating first layer 1 below second layer 2, with difference in level between the structures, whereby the tape is foldable into the half and windable into a roll shape.

Fig. 5 shows a structure that adhesive layer 3 is provided below almost the half width of support A composed of first layer 1 and second layer 2 and release parting layer 4 is provided on the support; the remaining half takes a symmetric structure to the above structure.

Fig. 6 shows an adhesive tape using support A of 3 layered structure comprising first layer 1 having provided second layer 2 on both surfaces thereof.

Fig. 7 shows a structure that adhesive layer 3 is provided via polyethylene film 6 on one surface of support A of a structure comprising first layer 1 having laminated therebeneath nonwoven fabric 5.

Fig. 8 is an explanatory illustration showing a method for determining crosswise compression strength later described.

Furthermore, the first layer may also be partially provided onto the second layer in an uneven state, stripe-like state or lattice-like state, while it is not shown.

The adhesive tape of the present invention can be prepared by co-extrusion, extrusion coating, lamination with heat or adhesives, solvent coating of the respective layers.

By constructing support in the laminate structure of the first layer and the second layer, the adhesive tape of the present invention possesses softness, is gentle to the skin but does not injure the skin even when contacted with the skin, and is excellent in handling and processing properties because of appropriate softness and elasticity. Further, by disposing the first layer on the surface, the tape is also advantageous that its feeling is excellent and it is easy to sieze the tape due to large surface friction.

Hereafter the present invention is described in detail by referring to the examples below.

Examples 1 through 12

Elastic films and plastic films were prepared using materials shown in Table 1 as the first and second layers, respectively. The both films were subjected to two-coat coextrusion at 200°C through a T die to give a dual layer film.

Then an adhesive layer was provided on the plastic film side of the dual layer film to give an adhesive tape.

Further in the case of using nonwoven fabric, the first layer is formed onto nonwoven fabric by extrusion coating and an adhesive layer was provided on the other surface of nonwoven fabric to give an adhesive tape.

Comparative Examples 1 through 4

Adhesive tapes were prepared in a manner similar to the Examples except for using no first layer.

Properties of the tapes obtained in Examples and Comparative Examples were evaluated by the following. The results are shown in Table 2.

[Crosswise Compression Strength]

As shown in Fig. 8, dual layer film A (65 mm x 25 mm) of the support was wound up into the length direction to form a crepe (diameter of 20 mm). Such a crepe was put on a stand and compressed from above at 23°C and 65% RH under 10 mm/min to determine the maximum compression strength, when the crepe began to collapse. The maximum strength was divided by the thickness of the support to obtain the crosswise compression strength.

[Elasticity, softnesss, touch and easy seizure]

Diapers using the adhesive tape as fastener fixing tapes for paper diapers were used for 20 infants. Organoleptic evaluation was performed by the following criteria.

|  | Number of infants who feel unpleasant/20 |
|---|---|
| ◎ | 0/20 |
| o | 1 to 5/20 |
| △ | 6 to 10/20 |
| x | more than 11/20 |

[Rate of redness]

When diapers using the adhesive tape as fastener fixing tape for paper diapers were used for 20 infants, a rate of redness was measured.

[Total evaluation]

Total evaluation was performed by the above evaluation results.

Table 1 (parts by weight unless otherwise indicated)

| Material | Example | | | | | | | | | | | | Comparative Example | | | |
|---|---|---|---|---|---|---|---|---|---|---|---|---|---|---|---|---|
| Elastic film: | 1 | 2 | 3 | 4 | 5 | 6 | 7 | 8 | 9 | 10 | 11 | 12 | 1 | 2 | 3 | 4 |
| (a) Ethylenepropylene elastomer | 100 | - | - | - | - | - | - | - | - | - | - | - | - | - | - | - |
| (b) Styrene-butadiene block copolymer | - | 100 | - | - | - | - | - | - | - | - | - | - | - | - | - | - |
| (c) Styrene-ethylene/ butylene-styrene block copolymer $C_1$ | - | - | - | - | - | 100 | 100 | - | - | - | - | - | - | - | - | - |
| $C_2$ | - | - | - | - | - | - | - | 100 | 100 | - | - | 100 | - | - | - | - |
| (d) Polyester type elastomer | - | - | 100 | - | - | - | - | - | - | - | - | - | - | - | - | - |
| (e) Urethane type elastomer | - | - | - | 100 | - | - | - | - | - | - | - | - | - | - | - | - |
| (f) Vinyl chloride type elastomer | - | - | - | - | 100 | - | - | - | - | - | - | - | - | - | - | - |
| (g) Polyethylene | - | - | - | - | - | 100 | 50 | 100 | - | - | - | 100 | - | - | - | - |
| (h) Polypropylene | - | - | - | - | - | - | 50 | - | 50 | - | - | - | - | - | - | - |
| (i) Ethylene-vinyl acetate copolymer | - | - | - | 50 | 50 | - | - | - | - | 100 | - | - | - | - | - | - |
| (j) Low density Ultrapolyethylene | - | 30 | - | - | - | - | - | - | - | - | 100 | - | - | - | - | - |
| Thickness (μm) | 100 | 100 | 100 | 100 | 100 | 50 | 50 | 50 | 50 | 50 | 50 | 50 | - | - | - | - |
| Plastic film: | | | | | | | | | | | | | | | | |
| (k) Polyethylene | 50 | 50 | - | - | - | 100 | 100 | - | 100 | 100 | 100 | - | 50 | 95 | - | - |
| (l) Ethylene-vinyl acetaet copolymer | - | - | - | 90 | 90 | - | - | 50 | - | - | - | - | - | - | - | - |
| (m) Ethylene-propylene copolymer | - | - | - | - | - | 10 | - | - | - | - | - | - | - | 40 | 10 | - |
| (n) Polypropylene | 50 | 50 | 100 | 10 | 10 | 100 | 50 | 100 | 100 | 100 | 100 | - | 50 | 5 | 100 | 100 |
| Thickness (μm) | 200 | 200 | 200 | 200 | 200 | 200 | 200 | 200 | 200 | 200 | 200 | 200 | 200 | 200 | 150 | 200 |
| Nonwoven fabric (polyester type) (thickness 50 μm) | | | | | | | | | | | | Weight 50 | | | | |
| Adhesive layer: Adhesive type | styrene | | | | | rubber | | | | | | | styrene | | rubber | |
| Thickness (μm) | 50 | 50 | 50 | 50 | 50 | 50 | 50 | 50 | 50 | 50 | 50 | 50 | 50 | 50 | 50 | 50 |

Details of the materials shown in Table 1 are as follows.

(a) Ethylenepropylene elastomer (TPE 1500 manufactured by Sumitomo Chemical Industry Co., Ltd.)

Table 2

| Item Evaluated | Example | | | | | | | | | | | | Comparative Example | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 1 | 2 | 3 | 4 |
| Crosswise compression strength (g/mm$^2$) | 50 | 55 | 143 | 63 | 56 | 68 | 89 | 62 | 99 | 52 | 53 | 63 | 178 | 52 | 204 | 228 |
| Elasticity | O | O | O | O | O | O | O | O | O | O | O | O | O | X | X | O |
| Softness | O | O | O | O | O | O | O | O | O | O | O | ◎ | X | O | △ | X |
| Touch | O | O | O | O | O | O | O | O | O | O | O | O | △ | O | △ | X |
| Easiness to seize | O | O | O | O | O | O | O | O | O | O | O | O | X | X | X | X |
| Rate of redness to the skin (%) | 0 | 0 | 20 | 0 | 0 | 0 | 5 | 0 | 5 | 0 | 0 | 0 | 100 | 0 | 100 | 100 |
| Total evaluation | Excell-ent | Excell-ent | Good | Excell-ent | Excell-ent | Excell-ent | Good | Excel-ent | Good | Excell-ent | Excell-ent | Excell-ent | Poor | Poor | Poor | Poor |

7

| Permanent compression strain(%): | 51 |
|---|---|
| Shore hardness: | A61 |

(b) Styrene-butadiene type block copolymer (CALIFLEX TR-1102 manufactured by Shell Co., Ltd.)

| Permanent compression strain(%): | 30-50 |
|---|---|
| Shore hardness: | A62 |
| Weight ratio of styrene/butadiene: | 28/72 |

(c) Styrene-ethylene/butylene-styrene block copolymer
   $c_1$ (CREYTON G-1652 manufactured by Shell Co., Ltd.)

| Permanent compression strain(%): | 30-50 |
|---|---|
| Shore hardness: | A75 |
| Weight ratio of styrene/rubber: | 29/71 |

   $c_2$ (CREYTON G-1657 manufactured by Shell Co., Ltd.)

| Permanent compression strain(%): | 30-50 |
|---|---|
| Shore hardness: | A65 |
| Weight ratio of styrene/rubber: | 14/86 |

(d) Polyester type elastomer (P-40H manufactured by Toyobo Co., Ltd.)

| Permanent compression strain(%): | 50 |
|---|---|
| Shore hardness: | D38 |

(e) Urethane type elastomer (PANDEX T-5010 manufactured by Dainippon Ink Industries, Inc.)

| Permanent compression strain(%): | 30 |
|---|---|
| Shore hardness: | D53 |

(f) Vinyl chloride type elastomer (SUMIFLEX manufactured by Sumitomo Bakelite Co., Ltd.)

| Permanent compression strain(%): | 47 |
|---|---|
| Shore hardness: | A62 |

(g) Polyethylene (NUCG-5220 manufactured by Nippon Unica Co., Ltd.)

| MI (dg/min): | 2.0 |
|---|---|
| Density ($g/m^2$): | 0.919 |

(h) Polypropylene (HI-POL B-230 manufactured by Mitsui Petrochemical Co., Ltd.)

| MFR (g/10 min): | 0.5 (ASTM D1238) |
|---|---|
| Density ($g/m^2$): | 0.91 |

(i) Ethylene-vinyl acetate copolymer (EVAFLEX EV-360 manufactured by Mitsui Petrochemical Co., Ltd.)

8

| Vinyl acetate content: | 25% |
|---|---|
| MI (dg/min): | 2.0 |
| Density (g/m$^2$): | 0.94 |

(j) Ultra low density polyethylene (YUKALON SELL X-139 manufactured by Mitsubishi Petrochemical Co., Ltd.)

| MFR (g/10 min): | 2 (JIS K6760) |
|---|---|
| Density (g/m$^2$): | 0.90 |

(k) Polyethylene (NUCG-5220 manufactured by Nippon Unica Co., Ltd.)

| MI (dg/min): | 2.0 |
|---|---|
| Density (g/m$^2$): | 0.919 |

(l) Ethylene-vinyl acetate copolymer (V-141 manufactured by Nippon Petrochemical Co., Ltd.)

| MFR (g/10 min): | 0.3 (JIS K6760) |
|---|---|
| Density (g/m$^2$): | 0.929 |

(m) Ethylenepropylene copolymer (SOFTREX C-9001 manufactured by Nippon Petrochemical Co., Ltd.)

| MFR (g/10 min): | 0.5 ( ASTM D1238 ) |
|---|---|
| Density (g/m$^2$): | 0.90 |

(n) Polypropylene
  same as (h)

## Claims

1.  A medical or sanitary adhesive tape comprising a support of a laminate structure composed of a first layer containing a thermoplastic elastomer and a second layer comprising a plastic film or nonwoven fabric, and an adhesive layer formed on at least one surface of said support, characterized in that a crosswise compression strength of said support is not greater than 200 g/mm$^2$ and said thermoplastic elastomer used in the first layer has a permanent compression strain of 5 to 100% and a Shore hardness of not less than A20 and not more than D70.

2.  A medical or sanitary adhesive type as claimed in claim 1, wherein said first layer is an elastic film mainly composed of one of or a mixture of at least two thermoplastic elastomer(s) selected from polyolefin type, polystyrene type, polyvinyl chloride type, polyester type, polyurethane type and chlorinated polyethylene type.

3.  A medical or sanitary adhesive tape as claimed in claim 1, wherein said first layer is an elastic film composed of a thermoplastic elastomer and one of or a mixture of at least two thermoplastic elastomer(s) selected from polyethylene, polypropylene or ethylene-vinyl acetate copolymer.

4.  A medical or sanitary adhesive tape as claimed in claim 1, wherein said first layer is an elastic film composed of the hydrogenated product of a block copolymer represented by general formula:

    A - (B - A)n

    wherein A represents a polymer block of monovinyl-substituted aromatic hydrocarbon; B represents an elastomeric polymer block of conjugated diene and n represents 1 to 4.

9

5. A medical or sanitary adhesive tape as claimed in claim 1, wherein said first layer is an elastic film composed of a mixture of the hydrogenated product of a block copolymer represented by general formula:

A - (B - A)n

wherein A represents a polymer block of monovinyl-substituted aromatic hydrocarbon; B represents an elastomeric polymer block of conjugated diene and n represents 1 to 4, and polyolefin type thermoplastic resin.

6. A medical or sanitary adhesive tape as claimed in claim 1, wherein said second layer is a plastic film comprising a mixture of at least one thermoplastic resin selected from polyethylene, ethylene-propylene copolymer and ethylene-vinyl acetate copolymer and polypropylene, or comprising polypropylene alone.

7. A medical or sanitary adhesive tape as claimed in claim 1, wherein said second layer is nonwoven fabric composed of polyester type, nylon type or polyolefin type.

8. A medical or sanitary adhesive tape as claimed in claim 1, wherein an adhesive layer is provided on the second layer side of the support.

9. A medical or sanitary adhesive tape as claimed in claim 1, wherein a release parting layer is further provided on the side opposite the adhesive layer of the support.

**Patentansprüche**

1. Medizinischer oder sanitärer Klebestreifen, umfassend einen Träger mit Laminatstruktur, zusammengesetzt aus einer ersten Schicht, enthaltend ein thermoplastisches Elastomer, und einer zweiten Schicht, umfassend einen Kunststofffilm oder Vlies, und eine Klebstoffschicht, gebildet auf mindestens einer Oberfläche des Trägers, **dadurch gekennzeichnet**, daß die Kompressionsfestigkeit in Querrichtung des Trägers nicht größer als 200 g/mm$^2$ ist und das in der ersten Schicht verwendete thermoplastische Elastomer eine permanente Kompressionsverformung von 5 bis 100% und eine Shore-Härte von nicht weniger als A20 und nicht mehr als D70 besitzt.

2. Medizinischer oder sanitärer Klebestreifen nach AnSpruch 1, worin die erste Schicht ein elastischer Film ist, hauptsächlich zusammengesetzt aus einem oder einem Gemisch von mindestens zwei thermoplastischen Elastomer(en), ausgewählt vom Polyolefintyp, Polystyroltyp, Polyvinylchloridtyp, Polyestertyp, Polyurethantyp und vom Typ des chlorierten Polyethylens.

3. Medizinischer oder sanitärer Klebestreifen nach Anspruch 1, worin die erste Schicht ein elastischer Film ist, zusammengesetzt aus einem thermoplastischen Elastomer und einem oder einem Gemisch von mindestens zwei thermoplastischen Elastomer(en), ausgewählt aus Polyethylen, Polypropylen oder Ethylen-Vinylacetat-Copolymer.

4. Medizinischer oder sanitärer Klebestreifen nach Anspruch 1, worin die erste Schicht ein elastischer Film ist, zusammengesetzt aus dem hydrierten Produkt eines Blockcopolymers, dargestellt durch die allgemeine Formel:

A - (B - A)n

worin A einen Polymerblock eines monovinyl-substituierten aromatischen Kohlenwasserstoffs darstellt; B stellt einen elastomeren Polymerblock eines konjugierten Diens dar, und n bedeutet 1 bis 4.

5. Medizinischer oder sanitärer Klebestreifen nach Anspruch 1, worin die erste Schicht ein elastischer Film ist, zusammengesetzt aus einem Gemisch des hydrierten Produktes eines Blockcopolymers, dargestellt durch die allgemeine Formel:

A - (B - A)n

worin A einen Polymerblock eines monovinyl-substituierten aromatischen Kohlenwasserstoffs darstellt; B stellt einen elastomeren Polymerblock eines konjugierten Diens dar, und n bedeutet 1 bis 4, und einem thermoplastischen Harz vom Polyolefintyp.

6. Medizinischer oder sanitärer Klebestreifen nach Anspruch 1, worin die zweite Schicht ein Kunststofffilm ist, umfassend ein Gemisch von mindestens einem thermoplastischen Harz, ausgewählt aus Polyethylen, Ethylen-Propylen-Copolymer und Ethylen-Vinylacetat-Copolymer und Polypropylen, oder umfassend Polypropylen alleine.

7. Medizinischer oder sanitärer Klebestreifen nach Anspruch 1, worin die zweite Schicht Vlies ist, zusammengesetzt aus Vlies vom Polyestertyp, Nylontyp oder Polyolefintyp.

8. Medizinischer oder sanitärer Klebestreifen nach Anspruch 1, worin auf der Seite der zweiten Schicht des Trägers eine Klebstoffschicht aufgebracht ist.

9. Medizinischer oder sanitärer Klebestreifen nach Anspruch 1, worin weiterhin eine Trennschicht auf der der Klebstoffschicht des Trägers gegenüberliegenden Seite aufgebracht ist.

**Revendications**

1. Un ruban adhésif à usage médical ou hygiénique comprenant un support présentant une structure stratifiée composée d'une première couche contenant un élastomère thermoplastique et d'une seconde couche composée d'un film plastique ou d'un tissu non tissé, et une couche adhésive formée sur au moins une surface dudit support, caractérisé en ce qu'une résistance à la compression transversale dudit support n'excède pas 200 g/mm$^2$ et que ledit élastomère thermoplastique utilisé dans la première couche présente une déformation sous pression permanente allant de 5 à 100 %, ainsi qu'une dureté Shore ne se situant pas en deça de A20 et pas au delà de D70.

2. Un ruban adhésif à usage médical ou hygiénique selon la Revendication 1, dans lequel ladite première couche est un film élastique composé essentiellement d'un élastomère thermoplastique ou d'un mélange d'au moins deux élastomères thermoplastiques, sélectionné(s) parmi ceux de type polyoléfine, polystyrène, chlorure de polyvinyle polyester, polyuréthane et un type polyéthylène chlore.

3. Un ruban adhésif à usage médical ou hygiénique selon la Revendication 1, dans lequel ladite première couche est un film élastique composé d'un élastomère thermoplastique et d'un ou d'un mélange d'au moins deux élastomères thermoplastiques sélectionnés parmi le polypropylène, le polyéthylène et un copolymère d'éthylène-acétate de vinyle.

4. Un ruban adhésif à usage médical ou hygiénique selon la Revendication 1, dans lequel ladite première couche est un film élastique composé du produit hydrogéné d'un copolymère bloc représenté par la formule générale suivante :

A-(B-A)n

dans laquelle A correspond à un polymère bloc d'hydrocarbure aromatique de monovinyle substitué ; B correspond à un polymère bloc élastomère de diènes conjugués, et n est un entier de 1 à 4.

5. Un ruban adhésif à usage médical ou hygiénique selon la Revendication 1, dans lequel ladite première couche est un film élastique composé du mélange d'un produit hydrogéné du copolymère bloc représenté par la formule générale suivante :

A-(B-A)n

dans lequel A correspond à un polymère bloc d'hydrocarbure aromatique de monovinyle substitué ; B correspond à un polymère bloc élastomère de diène conjugué, et n est un entier de 1 à 4, et d'une résine thermoplastique de type polyoléfine.

6. Un ruban adhésif à usage médical ou hygiénique selon la Revendication 1, dans lequel ladite seconde couche est un film plastique composé d'un mélange d'au moins une résine thermoplastique sélectionnée parmi le polyéthylène, un copolymère éthylène-propylène, un copolymère d'éthylène-acétate de vinyle, et le polypropylène, ou comprenant du polypropylène seul.

7. Un ruban adhésif à usage médical ou hygiénique selon la Revendication 1, dans lequel ladite seconde couche est un tissu non tissé de type polyester, nylon ou polyoléfine.

8. Un ruban adhésif à usage médical ou hygiénique selon la Revendication 1, dans lequel une couche adhésive est fournie sur la face de la seconde couche du support.

9. Un ruban adhésif à usage médical ou hygiénique selon la Revendication 1, dans lequel une couche d'arrêt à détacher est en outre fournie sur la face opposée à la couche adhésive du support.

# FIG. 1

4 RELEASE PARTING LAYER

1 FIRST LAYER } A SUPPORT
2 SECOND LAYER }

3 ADHESIVE LAYER

# FIG. 2

# FIG. 3

# FIG. 4

13

## FIG. 5

## FIG. 6

## FIG. 7

1 — FIRST LAYER
5 — NONWOVEN FABRIC
6 — POLYETHYLENE FILM

## FIG. 8